(19) Europäisches Patentamt
European Patent Office
Office européen des brevets

(11) Veröffentlichungsnummer: **0 051 791**
**A1**

(12) # EUROPÄISCHE PATENTANMELDUNG

(21) Anmeldenummer: **81108956.4**

(22) Anmeldetag: **27.10.81**

(51) Int. Cl.[3]: **C 07 C 49/457**, C 07 C 49/517, C 07 C 69/14, C 07 C 69/16, C 07 C 69/63, C 07 C 103/42, C 07 C 121/46, C 07 C 147/02, C 07 C 147/06, C 07 C 153/09, C 07 F 9/40 // C07C62/02, C07C62/08, C07C69/757, C07C51/09,

(30) Priorität: **08.11.80 DE 3042218**

(43) Veröffentlichungstag der Anmeldung: **19.05.82 Patentblatt 82/20**

(84) Benannte Vertragsstaaten: **CH DE FR GB IT LI**

(71) Anmelder: **BAYER AG, Zentralbereich Patente, Marken und Lizenzen, D-5090 Leverkusen 1, Bayerwerk (DE)**

(72) Erfinder: **Heine, Hans-Georg, Dr., Am Heckerhof 14, D-4150 Krefeld (DE)**
Erfinder: **Hartmann, Willy, Dr., Hohenzollernstrasse 25, D-4150 Krefeld (DE)**

(54) **2,2-Dimethylcyclobutanone und Verfahren zu ihrer Herstellung.**

(57) 2,2-Dimethylcyclobutanone der Formel I

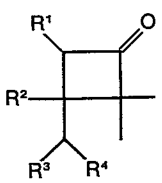

worin

$R^1$ für Wasserstoff, Chlor oder Brom,
$R^2$ für Wasserstoff, Methyl oder Phenyl,
$R^3$ für Wasserstoff, Alkyl, Aralkyl, Aryl, Halomethyl, Acyloxymethyl oder den Rest P(O) (O Alkyl)$_2$ steht und
$R^4$ für Chlor, Brom, Cyan, Alkoxy, Aryloxy, Acyloxy, Acylmercapto, Arylsulfonyl, Alkylsulfonyl, Acylamino oder P(O) (O Alkyl)$_2$ steht,

sind Zwischenprodukte für Dimethylcyclopropancarbonsäure-Derivate.

Man erhält sie, wenn man terminale Olefine der Formel II

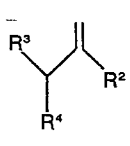

(II)

mit 1-Chlor-1-dimethylamino-2-methyl-propen-1 und einer Lewissäure, vorzugsweise Zinkchlorid, umsetzt und das Reaktionsprodukt anschließend hydrolysiert und gegebenenfalls mit einem Halogenierungsmittel umsetzt.

EP 0 051 791 A1

0051791

BAYER AKTIENGESELLSCHAFT 5090 Leverkusen, Bayerwerk

Zentralbereich
Patente, Marken und Lizenzen Rt-by-c
Ia/ZP

2,2-Dimethylcyclobutanone, und Verfahren zu ihrer Herstellung

Die vorliegende Erfindung betrifft neue 2,2-Dimethylcyclobutanone, sowie ein Verfahren zu ihrer Herstellung.

Geminale Dimethylcyclobutanone sind interessante Zwischenprodukte für die Herstellung von Dimethylcyclopropancarbonsäure-Derivaten. So ist z.B. bekannt geworden, 2,2-Dimethyl-3-(ß,ß-dichlor-vinyl)-cyclobutanon in 4-Stellung zu halogenieren und das resultierende Haloketon durch Behandeln mit 2n NaOH in Permethrinsäure umzuwandeln (DOS 2 650 534), deren Phenoxybenzylester eine starke Wirkung als Insektizid zeigt. Die bis jetzt bekannten Cyclobutanone führten nicht zu dem ebenfalls zur Herstellung guter Insektizide verwendbaren 2,2-Dimethyl-3-hydroxymethylcyclopropancarbonsäureester. Dieser wurde bis jetzt in einer Wittig Reaktion

aus Fumarsäureester mit Isopropylidentriphenylphosphoran über den Cyclopropandicarbonsäureester, der partiell hydrolysiert und reduziert wurde, erhalten. Dabei beträgt die Gesamtausbeute nur ca. 35 %, was das Verfahren in Anbetracht der teuren Ausgangsverbindungen unwirtschaftlich macht (Tetrahedron Letters 1978, S. 1847 ff).

Es wurden neue 2,2-Dimethylcyclobutanone der Formel I

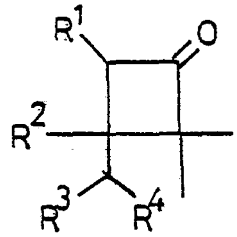

(I)

gefunden, worin

$R^1$ für Wasserstoff, Chlor oder Brom,

$R^2$ für Wasserstoff, Methyl oder Phenyl,

$R^3$ für Wasserstoff, niederes Alkyl, gegebenenfalls substituiertes Aralkyl oder Aryl, Halomethyl, Acyloxymethyl oder den Rest $P(O)(o\text{-niederes Alkyl})_2$ steht und

$R^4$ für Halogen, Cyan, Alkoxy, Aryloxy, Acyloxy, Acylmercapto, Arylsulfonyl, Alkylsulfonyl, Acylamino oder $P(O)(O\text{-niederes Alkyl})_2$ steht.

Weiterhin wurde gefunden, daß man die neuen 2,2-Dimethylcyclobutanone erhält, wenn man terminale Olefine der Formel II

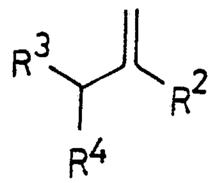

(II)

mit 1-Chlor-1-dimethylamino-2-methyl-propen-1 und einer Lewis-Säure, vorzugsweise Zinkchlorid, umsetzt und das Reaktionsprodukt anschließend hydrolysiert und gegebenenfalls mit einem Halogenierungsmittel umsetzt.

Mit Hilfe der neuen Verbindungen ist beispielsweise 2,2-Dimethyl-3-hydroxymethylcyclopropancarbonsäure in guter Ausbeute und ausgehend von preiswerten Ausgangsstoffen zugänglich.

Bevorzugt sind Verbindungen der Formel I in welcher

$R^1$ für Wasserstoff, Chlor oder Brom steht,

$R^2$ für Wasserstoff oder Methyl steht,

$R^3$ für Wasserstoff oder $P(O)(O-C_{1-4}-Alkyl)_2$ steht,

$R^4$ für Chlor, Brom, Cyan, Acetoxy, Acetylmercapto, $C_1$-$C_4$-Alkoxy, Acetylamino, gegebenenfalls $C_{1-4}$-alkylsubstituiertes Phenylsulfonyl oder $P(O)(O-C_{1-4}-Alkyl)_2$ steht.

Als Beispiele seien genannt:

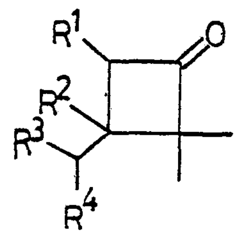

| $R^1$ | $R^2$ | $R^3$ | $R^4$ |
|---|---|---|---|
| H | H | H | Cl |
| Br | H | H | Cl |
| H | H | H | Br |
| Br | H | H | Br |
| H | H | H | $OCOCH_3$ |
| Cl | H | H | $OCOCH_3$ |
| Br | H | H | $OCOCH_3$ |
| H | H | H | $OCH_3$ |
| Br | H | H | $OCH_3$ |
| H | H | H | $NHCOCH_3$ |
| Br | H | H | $NHCOCH_3$ |
| H | H | H | $SCOCH_3$ |
| H | H | H | p-Tosyl |
| Cl | H | H | p-Tosyl |
| Br | H | H | p-Tosyl |
| H | H | H | CN |
| Br | H | H | CN |
| H | H | H | $P(O)\ (OCH_3)_2$ |
| H | H | H | $P(O)\ (OC_2H_5)_2$ |
| H | H | $P(O)\ (OCH_3)_2$ | $OCOCH_3$ |
| H | $CH_3$ | H | Cl |
| H | H | $CH_2OCOCH_3$ | $OCOCH_3$ |
| H | H | $CH_2Cl$ | Cl |
| Br | H | $CH_2OCOCH_3$ | $OCOCH_3$ |

Das Verfahren zur Herstellung der neuen 2,2-Dimethylcyclobutanone ist im Prinzip bekannt. Wegen niedriger Reaktionstemperaturen und einfacher Durchführbarkeit hat sich die Umsetzung von α-Chlorenaminen mit den Olefinen der Formel II bewährt.

Verwendet man beispielsweise Allylmethylether als Olefin, so kann der Reaktionsablauf durch folgendes Schema wiedergegeben werden:

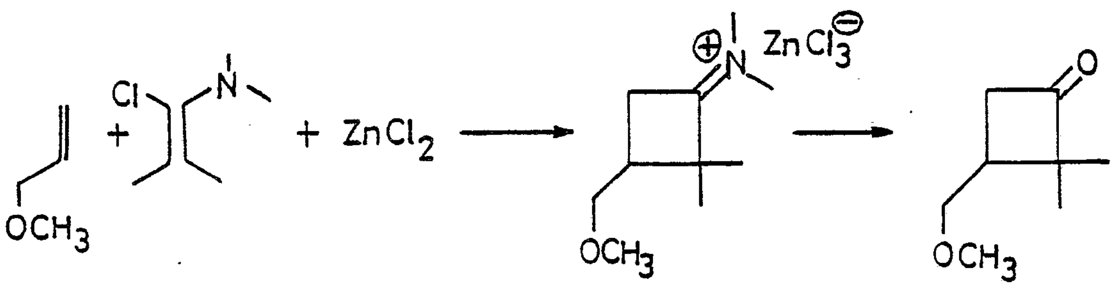

Es ist ausgesprochen überraschend, daß unter den angegebenen Reaktionsbedingungen keine Etherspaltung eintritt. Auch bei Verwendung solch reaktiver Verbindungen wie Allylchlorid oder Allylbromid als olefinische Substrate erfolgt keine Reaktion mit der Lewis-Säure Zinkchlorid.

Für das Verfahren ist es erforderlich, das α-Chlorenamin in eine reaktionsfähige Form zu überführen. Dies kann durch Reaktion des α-Chlorenamins beispielsweise mit Silbertetrafluoroborat erfolgen. Billiger für die Durchführung der Cycloaddition mit α-Chlorenaminen ist die Verwendung von Zinkchlorid. Bei der Addition des α-Chlorenamins an die Olefine der

Formel II erfolgt unter den angewendeten schonenden Reaktionsbedingungen (s. unten) keine Reaktion des Zinkchlorids mit dem Olefin der Formel II. Es lassen sich jedoch für die Umsetzung auch andere Lewis-Säuren wie z.B. Titantetrachlorid, Zinnchlorid, verwenden.

Die Umsetzung des α-Chlorenamins mit einem Olefin der Formel II kann so durchgeführt werden, daß das Olefin gegebenenfalls in einem Lösungsmittel zusammen mit einer Lewis-Säure vorgelegt wird und das α-Chlorenamin gegebenenfalls in einem Lösungsmittel unter Rühren zugetropft wird. Hierbei kann eine positive Wärmetönung auftreten. Es kann aber auch so verfahren werden, daß das α-Chlorenamin gegebenenfalls in einem Lösungsmittel vorgelegt wird, durch Zugabe einer Lewis-Säure das reaktive Ketenimoniumkation erzeugt und das Olefin gegebenenfalls in einem Lösungsmittel zugetropft wird. Auch hierbei kann eine positive Wärmetönung auftreten. Eine weitere Variante besteht darin, daß die Reaktionskomponenten ( α-Chlorenamin, Lewis-Säure, Olefin) gegebenenfalls in einem Lösungsmittel zusammengegeben und gerührt werden. Wiederum kann eine positive Wärmetönung auftreten. Schließlich besteht eine weitere Variante darin, das zu verwendende α-Chlorenamin, welches im allgemeinen stark hydrolyseempfindlich ist, in situ zu erzeugen und dann nach einem der skizzierten Wege umzusetzen.

Als Lösungsmittel können halogenierte Kohlenwasserstoffe, wie z.B. Methylenchlorid, Chloroform, Tetrachlorkohlenstoff, 1,2-Dichlorethan, oder Ether wie z.B. Diethylether, Tetrahydrofuran, oder Ester, wie z.B. Essigsäureethylester, oder Acetonitril, Kohlenwasserstoffe, wie z.B. Cyclohexan, Petrolether, eingesetzt werden.

Die Cycloaddition eines α-Chlorenamins an ein Olefin in Gegenwart einer Lewis-Säure ist eine stöchiometrisch ablaufende Reaktion. Es ist jedoch empfehlenswert, einen kleinen Überschuß an α-Chlorenamin und Lewis-Säure (max. ca. 20 %) zu wählen.

Die Reaktionstemperatur ist in einem weiten Bereich wählbar. So läßt sich die Umsetzung sowohl bei -10°C als auch bei +80°C durchführen. In vielen Fällen hat es sich gezeigt, daß die Cycloaddition bereits in dem technisch einfach zu bewältigenden Temperaturbereich von 20 bis 40°C, d.h. bei Raumtemperatur, oder wenig darüber, eintritt. Eine Reaktionszeit von 1/2 bis 24 Stunden ist für einen vollständigen Umsatz ausreichend.

Zur Aufarbeitung hydrolysiert man das Reaktionsgemisch des Verfahrens durch Zugabe von Wasser, einer wäßrigen Base oder Säure. Hierbei wird das intermediär gebildete Cyclobutanonimoniumsalz gegebenenfalls durch Erwärmen der Lösung auf Temperaturen

zwischen 20 und 100°C, vorzugsweise 40 bis 60°C, in das Cyclobutanonderivat der allgemeinen Formel I übergeführt, das durch Extrahieren mit einem organischen Lösungsmittel, wie z.B. Toluol oder Dibutylether, abgetrennt wird. Durch fraktionierendes Destillieren, gegebenenfalls unter vermindertem Druck, und/oder Kristallisieren kann es in analysenreiner Form zur Charakterisierung erhalten werden.

In den Fällen, in denen man Verbindungen der Formel I erhalten will, in der $R^1$ für Chlor oder Brom steht, kann das wie vorstehend beschrieben erhaltene rohe Cyclobutanon direkt in die Halogenierung eingesetzt werden.

Als Halogenierungsmittel können verwendet werden: Brom, Chlor, Sulfurylchlorid, Pyridiniumperbromid.

Als bevorzugte Verdünnungsmittel für die Durchführung der Halogenierung seien folgende inerte aprotische organische Lösungsmittel genannt: Ester von Mono- und Dicarbonsäuren, wie z.B. Essigsäureethylester, Essigsäuremethylester, Propionsäuremethylester, Bernsteinsäurediethylester, Ameisensäure-n-butylester, Ameisensäuremethylester, Ameisensäureethylester, Dimethylcarbonat, Bernsteinsäuredimethylester, oder Ether, wie z.B. Diethylether, Di-iso-propylether, Dioxan, Tetrahydrofuran, Dimethylether, Methylbutylether sowie Methylenchlorid, Chloroform oder Tetrachlorkohlenstoff.

Als Katalysatoren, die zur Durchführung gegebenenfalls verwendet werden können, seien bevorzugt Halogenwasserstoffsäuren wie Bromwasserstoff, Chlorwasserstoff, Tetrabutylammoniumbromid, Pyridiniumbromid, Pyridiniumchlorid genannt.

Die Reaktionstemperatur der Halogenierung ist in einem relativ weiten Bereich wählbar. Als präparativ nützlich erweist sich ein Temperaturbereich von -20° bis +60°, vorzugsweise 20 bis 50°.

Die Halogenierung wird bevorzugt so durchgeführt, daß das Cyclobutanon in einem Verdünnungsmittel gegebenenfalls zusammen mit einem Katalysator vorgelegt wird und das Halogenierungsmittel portionsweise in die Lösung eingetragen wird, wobei die Geschwindigkeit der Zugabe sich nach dem Umsatz des Halogenierungsmittels richtet, d.h. erst wenn zuvor eingetragenes Halogenierungsmittel umgesetzt ist, erfolgt die Zugabe einer weiteren Menge Halogenierungsmittel. Eine andere Methode besteht darin, daß man die Reaktanden (Cyclobutanonderivat und Halogenierungsmittel, Lösungsmittel und gegebenenfalls Katalysator) zusammengibt und bei 20 bis 50° reagieren läßt. Eine weitere Variante besteht darin, daß ein Teil des sich während der Reaktion bildenden Halogenwasserstoffs mit Stickstoff aus der Reaktionslösung ausgetrieben oder durch Umsetzung mit einer basischen Verbindung, wie z.B. Calciumcarbonat oder Soda, entfernt wird.

Die Aufarbeitung der Reaktionslösung kann so erfolgen, daß der Halogenwasserstoff mit Stickstoff oder Luft ausgetrieben wird und die Reaktionslösung gegebenenfalls nach Entfernen überschüssigen Halogenierungsmittels mit Natriumthiosulfat und ausgeschiedenen Hydrohalogenids der tert. Stickstoffbase direkt in die Umsetzung mit einer wäßrigen Alkalibase zu der entsprechenden Cyclopropancarbonsäure oder in die Umsetzung mit einem Alkalisalz eines niederen Alkohols wie Ethanol zu einem Cyclopropancarbonsäureester dieses niederen Alkohols eingesetzt wird. Das rohe -Halogenketon läßt sich durch Waschen der Reaktionslösung mit Wasser, gegebenenfalls unter Zusatz eines mit Wasser nicht mischbaren Lösungsmittels, halogenwasserstofffrei erhalten und durch Kristallisieren oder Destillieren in reiner Form isolieren.

Die neuen Dimethylcyclobutanone der Formel I stellen wie schon erwähnt, wertvolle Zwischenprodukte zur Herstellung von Insektiziden dar. Die Cyclobutanone sind auf diese Weise in einer Reaktion unter milden Bedingungen rein und in guten Ausbeuten, auch im technischen Maßstab zugänglich. So kann gemäß folgender Reaktionsschritte beispielsweise Caronaldehyd als Zwischenprodukt für Insektizide zugänglich gemacht werden.

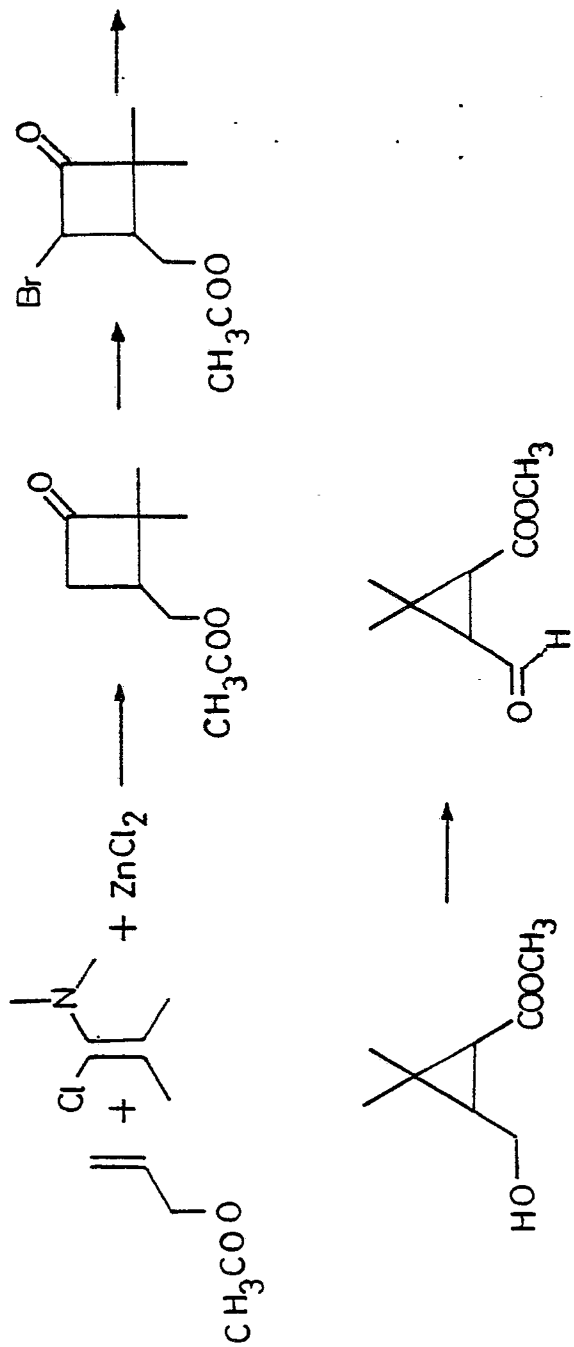
+ $ZnCl_2$

Die Oxidation des 2,2-Dimethyl-3-hydroxy-methyl-cyclopropancarbonsäureesters ist bekannt (Tetrahedron Letters 1978, 1847); beschrieben ist auch die Verwendung des Caronaldehyds für die Synthese von Pyrethroiden (Südafr. Pat. 1 446 309).

Beispiel 1

Herstellung des Amidchlorids des Isobuttersäuredimethylamids

In ein Rührgefäß, versehen mit Rührer, Rückflußkühler, Tropftrichter und Gaseinleitungsrohr, gibt man die Lösung von 345,0 g (3,0 mol) Isobuttersäuredimethylamid in 2000 ml trockenem Methylenchlorid und leitet unter Kühlen bei 0°C 330,0 g (3,3 mol) Phosgen unter Rühren ein. Man läßt die Lösung auf 20-25°C erwärmen und destilliert nach Stehen über Nacht (15-20 Stunden) nichtumgesetztes Phosgen zusammen mit etwa 1/3 des als Lösungsmittel verwendeten Methylenchlorids ab. Den Rückstand verdünnt man durch Zugabe von Methylenchlorid auf 2100 ml.

Beispiel 2

Herstellung von 3-Acetoxymethyl-2,2-dimethyl-cyclobutanon

a. Zu 700 ml der gemäß Beispiel 1 bereiteten Lösung des Amidchlorids des Isobuttersäuredimethylamids in Methylenchlorid tropft man unter Kühlen und Rühren bei 20°C 100,0 g (1,0 mol) Triethylamin in 200 ml Methylenchlorid und erhitzt anschließend 1 Stunde zum Rückfluß. Darauf fügt man bei 10°C 150,0 g (1,1 mol) wasserfreies Zink-

0051791

chlorid hinzu und tropft 90,0 g (0,9 mol) Allylacetat in 100 ml Methylenchlorid innerhalb von 60 min. in die Reaktionslösung. Nach 5-stündigem Erhitzen zum Rückfluß versetzt man sie mit 500 ml Wasser und rührt über Nacht (15 Stunden). Trennen der Phasen, Trocknen der org. Phase über Natriumsulfat und fraktionierendes Destillieren liefern als Vorlauf 13,2 g farbloses Öl, $Sdp_{14}$ 80-102°C, und als Hauptlauf 93,7 g 3-Acetoxymethyl-2,2-dimethylcyclobutanon als farbloses Öl, $Sdp_{13}$ 108-111°C $n_D^{20}$ 1.4448.

Ausbeute ca. 61 %.

| | | | |
|---|---|---|---|
| $C_9H_{14}O_3$ | Ber. | C 63.51 | H 8.29 |
| (170.2) | Gef. | 63.65 | 8.17 |

IR ($CCl_4$): 1750 und 1785 $cm^{-1}$

$^1$H-NMR ($CDCl_3$): = 4.29 d (2H, J = 7.5Hz),
3.50-2.78 m (2H),
2.78-2.30 m (1H),
2.1 s (3H),
1.25 s (3H) und
1.16 ppm s (3H).

b. Zu der Suspension von 150.0 g (1.1 mol) wasserfreiem Zinkchlorid in 500 ml trockenem Methylenchlorid werden bei 20°C unter Rühren 135.0 g

0051791

(1.01 mol) 1-Chlor-1-dimethylamino-2-methyl-propen-1 in 100 ml trockenem Methylenchlorid und darauf 90,0 g (0,9 mol) Allylacetat in 100 ml trockenem Methylenchlorid getropft. Nach 4-stündigem Sieden zum Rückfluß fügt man 500 ml Wasser zu und läßt über Nacht rühren. Man trennt die Phasen, extrahiert die wäßrige Phase viermal mit je 150 ml Methylenchlorid und trocknet die vereinigten organischen Phasen über wasserfreiem Natriumsulfat. Filtrieren und Einengen liefern 146.3 g hellbraunes Öl, das fraktionierend destilliert wird.

| | | | | |
|---|---|---|---|---|
| Fraktion 1 | Sdp.$_{20-14}$ | 27-98°C | 5,9 g | |
| Fraktion 2 | Sdp.$_{14}$ | 96-103°C | 4,2 g | $n_D^{20}$ 1.4415 |
| Fraktion 3 | Sdp.$_{14}$ | 105-110°C | 121,3 g | 1.4433 |
| Rückstand | | | 2,0 g | |

Gaschromatographische Analyse

Fraktion 3 98,6 % } 71 % 3-Acetoxymethyl-2,2-dimethyl-cyclobutanon
Fraktion 2 71,2 % }

Analog Beispiel 2b sind erhalten worden:

1. 3-Chlormethyl-2,2,3-trimethyl-cyclobutanon Sdp.$_{13}$ 86-87°C (kristallisierendes Öl).

2. 2,2-Dimethyl-3-(1-acetoxy-1-dimethylphosphonyl-methyl)-cyclobutanon, Sdp.$_{0,07}$ 115-120°C, $n_D^{20}$ 1,4585.

3. 2,2-Dimethyl-3-(1-acetoxy-1-diethylphosphonylmethyl)-cyclobutanon, Sdp. $_{0,07}$ 118-125°C, $n_D^{20}$ 1,4535.

Beispiel 3

Herstellung von 3-Chlormethyl-2,2-dimethyl-cyclobutanon.

Entsprechend Beispiel 2b werden 30,0 g (0,22 mol) wasserfreies Zinkchlorid in 100 ml Methylenchlorid bei 20°C mit 30,0 g (0,22 mol) 1-Chlor-1-dimethylamino-2-methylpropen-1 unter Rühren umgesetzt. Zu der Lösung tropft darauf 19,1 g (0,2 mol) Allylchlorid und erhitzt 4 Stunden zum Rückfluß. Nach dem Abkühlen hydrolysiert man 2 Stunden mit 100 ml Wasser bei 20°C und unterwirft das Gemisch der Wasserdampfdestillation. Man extrahiert das Destillat 3mal mit je 150 ml Methylenchlorid, trocknet die org. Phase und engt sie ein. Fraktionierendes Destillieren über eine 10 cm, Vigreuxkolonne liefert 25.8 g (79 %) 3-Chlormethyl-2,2-dimethyl-cyclobutanon als farbloses Öl vom Sdp.$_{13}$ 80-82°C $n_D^{20}$ 1.4587.

| | | | | |
|---|---|---|---|---|
| $C_7H_{11}ClO$ (146.6) | Ber. | C 57.34 | H 7.56 | Cl 24.19 |
| | Gef. | 57.5 | 7,95 | 23.5 |

IR ($CCl_4$): 1790 $cm^{-1}$

$^1$H-NMR ($CDCl_3$): = 3.78 d (2H, J ~ 7Hz),
3.50-2.0 m (3H),
1.31 s (3H) und
1.25 ppm s (3H).

Beispiel 4

Herstellung von 3-Brommethyl-2,2-dimethyl-cyclobutanon

Entsprechend Beispiel 2b werden 30,0 g (0,22 mol) wasserfreies Zinkchlorid in 100 ml Methylenchlorid mit 30,0 g (0,22 mol) 1-Chlor-1-dimethylamino-2-methylpropen-1 in 30 ml Methylenchlorid umgesetzt. Zu dieser Lösung werden innerhalb von 5 min. 30,25 g (0,25 mol) Allylbromid bei 20°C zugetropft. Nach 5,5 Stunden intensiven Rührens unter Rückfluß wird die Lösung auf 20°C abgekühlt und mit 200 ml Wasser 2 Stunden bei 20°C gerührt. Das Gemisch unterwirft man der Wasserdampfdestillation und extrahiert das Destillat 3mal mit je 150 ml Methylenchlorid. Trocknen über wasserfreiem Natriumsulfat und Einengen liefern 39,4 g Rohprodukt, dessen fraktionierende Destillation 33,6 g 3-Brommethyl-2,2-dimethyl-cyclobutanon als helles Öl ergibt.
$Sdp._{13-12}$ 94-98°C $n_D^{20}$ 1.486.

| | | | | |
|---|---|---|---|---|
| $C_7H_{11}BrO$ (191.1) | Ber. | C 44.00 | H 5.80 | Br 41.83 |
| | Gef. | 44.3 | 5.92 | 40.2 |

IR ($CCl_4$): 1790 cm-1

$^1$H-NMR ($CDCl_3$): $\delta$ = 3,75 m (2H),
3,5-2,5 m (3H),
1,3 s (3H) und
1,22 ppm s (3H).

Beispiel 5

Herstellung von 3-Cyanmethyl-2,2-dimethyl-cyclobutanon

Entsprechend Beispiel 2b werden 30,0 g (0,22 mol) Zinkchlorid mit 30,0 g (0,22 mol) 1-Chlor-1-dimethylamino-2-methylpropen-1 in 130 ml Methylenchlorid bei 20°C unter Rühren umgesetzt. Anschließend gibt man innerhalb von 10 min. 14,0 g (0,2 mol) Allylcyanid hinzu und erhitzt 6 Stunden zum Rückfluß. Die halbfeste Masse (teilweise ist Polymerisation eingetreten) läßt man über Nacht bei 20°C stehen, fügt dann 200 ml 2 n NaOH hinzu und hydrolysiert 4 Stunden bei 40°C. Man stellt die Lösung mit 10 proz. Salzsäure sauer und arbeitet wie üblich auf. Fraktionierende Destillation des Rohprodukts (28,2 g) ergibt 10,5 g 3-Cyanmethyl-2,2-dimethyl-cyclobutanon als farbloses Öl vom $Sdp._{15}$ 137-139°C $n_D^{20}$ 1.4550.

| | | | | |
|---|---|---|---|---|
| $C_8H_{11}NO$ (137.2) | Ber. | C 70,05 | H 8,08 | N 10,21 |
| | Gef. | 70,1 | 8,08 | 10,15 |

IR ($CCl_4$): 1790 und 2250 $cm^{-1}$

$^1$H-NMR ($CDCl_3$): $\delta$ = 3,50-2,50 m (5H),
1,28 s (3H) und
1,12 ppm s (3H).

Beispiel 6

Herstellung von 3-(p-Tosylmethyl)-2,2-dimethyl-cyclobutanon

14,7 g (0,11 mol) 1-Chlor-1-dimethylamino-2-methyl-

 0051791

propen-1 in 50 ml Methylenchlorid werden bei 20°C unter Rühren mit 19,6 g (0,1 mol) p-Tolylallylsulfon und 16,5 g (0,12 mol) wasserfreiem Zinkchlorid in 125 ml Methylenchlorid umgesetzt. Nach 6-stündigem Rühren unter Rückfluß setzt man der Reaktionslösung 300 ml Wasser zu und rührt über Nacht bei 20°C. Aufarbeitung durch Extrahieren mit Methylenchlorid liefert 28,7 g Rohprodukt, dessen $^1$H-NMR-Spektrum ($CDCl_3$) außer Signalen von geringen Mengen an Edukten nur solche des Cycloaddukts zeigt. Kristallisieren aus Benzol/Petrolether liefert 3-(p-Tosylmethyl)-2,2-dimethyl-cyclobutanon als farblose Kristalle vom Schmp. 127-128°C.

| | | | | |
|---|---|---|---|---|
| $C_{14}H_{18}SO_3$ (266,4) | Ber. | C 63,13 | H 6,81 | S 12,04 |
| | Gef. | 63,15 | 6,51 | 12,0 |

IR ($CCl_4$): 1795 $cm^{-1}$

$^1$H-NMR ($CDCl_3$): $\delta$ = 7,6-8,0 m (4H),
3,5-3,0 m (5H),
2,6 s (3H),
1,25 s (3H) und
1,13 ppm s (3H).

<u>Beispiel 7</u>

Herstellung von 3-Methoxymethyl-2,2-dimethyl-cyclobutanon

Gemäß Beispiel 2b werden 30,0 g (0,22 mol) 1-Chlor-1-dimethylamino-2-methylpropen-1 bei 20°C in 30 ml Methylenchlorid zu der Suspension von 30,0 g (0,22 mol) Zinkchlorid in 100 ml Methylenchlorid getropft. Anschließend gibt man innerhalb von 5 min. 15,0 g (0,2 mol) Methylallylether zu und kocht 6 Stunden zum Rückfluß. Nach Stehen über Nacht hydrolysiert man mit 200 ml 2n NaOH unter Rühren bei 40°C, stellt mit 10 %iger Salzsäure sauer, trennt die Phasen und arbeitet die org. Phase auf. Nach Einengen im Vakuum werden 46,7 g Rohprodukt erhalten, die fraktionierend an einer 10 cm Vigreux-Kolonne destilliert werden. Ausbeute 21,5 g (75,6 %), Sdp.$_{13}$ 70-71,5°C $n_D^{20}$ 1.4343

| | | | |
|---|---|---|---|
| $C_8H_{14}O_2$ (142.2) | Ber. | C 67,57 | H 9,92 |
| | Gef. | 67,4 | 10,2 |

IR ($CCl_4$): 1785 $cm^{-1}$

$^1$H-NMR ($CDCl_3$): = 3,63-3,56 m (2H),
3,41 s (3H),
3,25-2,82 m (2H),
2,78-2,19 m (1H),
1,25 s (3H) und
1,16 ppm s (3H).

Beispiel 8

Herstellung von 3-Acetylmercaptomethyl-2,2-dimethylcyclobutanon

Zu dem Gemisch von 34,8 g (0,3 mol) Allylthioacetat und 50,0 g (0,36 mol) wasserfreiem Zinkchlorid in 450 ml Methylenchlorid werden unter Rühren innerhalb von 40 min. bei 25°C 44,1 g (0,33 mol) 1-Chlor-1-dimethylamino-2-methylpropen-1 in 100 ml Methylenchlorid zugetropft. Man erhitzt 6,5 Stunden zum Rückfluß, setzt 400 ml Wasser zu und rührt über Nacht. Extrahieren mit Methylenchlorid liefert 57,9 g rohes Cyclobutanon-Derivat, das fraktionierend destilliert wird. Man erhält 44,8 g (ca. 80 %) 3-Acetylmercaptomethyl-2,2-dimethyl-cyclobutanon als farbloses Öl vom $Sdp._{13}$ 134°C $n_D^{20}$ 1.4923.

| | | | | |
|---|---|---|---|---|
| $C_9H_{14}SO_2$ (186.3) | Ber. | C 58,03 | H 7,58 | S 17,21 |
| | Gef. | 58,3 | 7,56 | 17,0 |

IR ($CCl_4$): 1690 und 1775 $cm^{-1}$

$^1$H-NMR ($CDCl_3$) : $\delta$ = 3,4-2,4 m (5H),
2,36 s (3H),
1,24 s (3H) und
1,19 ppm s (3H).

Beispiel 9

Herstellung von 3-Acetylaminomethyl-2,2-dimethyl-cyclobutanon

Gemäß Beispiel 8 werden 29,7 g (0,3 mol) Allylacetamid und 50,0 g (0,36 mol) wasserfreies Zinkchlorid in 450 ml

Methylenchlorid mit 44,1 g (0,33 mol) 1-Chlor-1-dimethylamino-2-methylpropen-1 umgesetzt. Nach 6-stündigem Erhitzen zum Rückfluß setzt man 250 ml 2n NaOH zu, rührt über Nacht bei 20°C, säuert mit 50 ml konz. Salzsäure an und extrahiert mit Methylenchlorid. Den Rückstand (11,2 g) kristallisiert man aus Ethanol/Petrolether um und erhält 3-Acetylaminomethyl-2,2-dimethyl-cyclobutanon vom Schmp. 45-48°C.

| | | | | |
|---|---|---|---|---|
| $C_9H_{15}NO_2$ (169.2) | Ber. | C 63,88 | H 8,93 | N 8,28 |
| | Gef. | 64,1 | 8,59 | 8,11 |

IR ($CCl_4$): 1680 und 1770 $cm^{-1}$

$^1$H-NMR ($CDCl_3$): $\delta$ = 7,05 m (1H)
3,5-3,3 m (2H),
3,2-2,1 m (3H),
1,97 s (3H),
1,21 s (3H) und
1,13 ppm s (3H).

Beispiel 10

Herstellung von 3-($\alpha$,ß-dichlorethyl)-2,2-dimethylcyclobutanon

37,5 g (0,3 mol) 3,4-Dichlorbuten-1 und 50,0 g (0,36 mol) wasserfreies Zinkchlorid werden in 450 ml Methylenchlorid vorgelegt und innerhalb von 40 min. mit der

Lösung von 44,1 g (0,33 mol) 1-Chlor-1-dimethylamino-2-methylpropen-1 in 100 ml Methylenchlorid bei 20°C versetzt. Man erhitzt 7 Stunden zum Rückfluß, fügt 400 ml Wasser zu, rührt 2 Stunden nach und unterwirft die Reaktionslösung der Wasserdampfdestillation. Extrahieren der Destillate mit Methylenchlorid liefert 54,3 g gelbes Öl, das fraktionierend destilliert wird. Neben 10,1 g nicht umgesetztem Olefin erhält man 27,0 g 3-(α,ß-Dichlorethyl)-2,2-dimethyl-cyclobutanon als farbloses Öl vom Sdp.$_{0,07}$ 69-70°C $n_D^{20}$ 1.4856.

| | | | | |
|---|---|---|---|---|
| $C_8H_{12}Cl_2O$ (195.1) | Ber. | C 49,25 | H 6,20 | Cl 36,4 |
| | Gef. | 49,5 | 6,25 | 33,5 |

IR ($CCl_4$): 1795 $cm^{-1}$.

Beispiel 11

Herstellung von 3-(α,ß-Diacetoxyethyl)-2,2-dimethyl-cyclobutanon

17,2 g (0,1 mol) 3,4-Diacetoxybuten-1 und 16,5 g (0,12 mol) wasserfreies Zinkchlorid werden in 200 ml Methylenchlorid vorgelegt und mit 15,0 g (0,11 mol) 1-Chlor-1-dimethylamino-2-methylpropen-1 in 25 ml Methylenchlorid, wie für Beispiel 8 beschrieben, umgesetzt. Man erhält nach Hydrolyse 25,0 g farbloses Öl, dessen Destillation 3-(α,ß-Diacetoxyethyl)-2,2-dimethyl-cyclobutanon

vom $Sdp._{0,09-0,12}$ 101-104°C $n_D^{20}$ 1.4752 liefert.

| | | | |
|---|---|---|---|
| $C_{12}H_{18}O_5$ | Ber. | C 59,49 | H 7,49 |
| (242.3) | Gef. | C 59,1 | H 7,50 |

IR ($CCl_4$): 1750 und 1790 $cm^{-1}$.

Beispiel 12

Herstellung von 3-Acetoxymethyl-4-brom-2,2-dimethylcyclobutanon

a) 85,1 g (0,5 mol) 3-Acetoxymethyl-2,2-dimethylcyclobutanon werden in 750 ml Essigsäureethylester bei 20°C portionsweise mit insgesamt 159,5 g (0,5 mol) Pyridiniumperbromid unter Rühren versetzt. Nach Rühren über Nacht wird die schwach gelb gefärbte Lösung, aus der Pyridiniumbromid ausgefallen ist, mit Eiswasser versetzt. Man trennt die Phasen, ethert die wäßrige Phase aus, vereinigt die organischen Extrakte und trocknet sie. Fraktionierendes Destillieren des Rückstands (123.1 g) liefert 0,98 g Vorlauf (Edukt), 111,7 g 3-Acetoxymethyl-4-brom-2,2-dimethylcyclobutanon vom $Sdp._{0,11-0,18}$ 88-95°C (Stereoisomere im Verhältnis 9:1) sowie 3,4 g Rückstand (= 90 % Ausbeute).

$C_9H_{13}BrO_3$ Ber. C 43,39 H 5,26 Br 32,08
(249.1) Gef. 43,9 5,6 31,2

IR ($CCl_4$): 1745 und 1795 $cm^{-1}$

$^1$H-NMR ($CDCl_3$): $\delta$ = 5,25 d (J = 10 Hz) und 4,90 d (J = 8 Hz) (zusammen 1 H), 4,55-4,15 m (2 H), 3,25-2,50 m (1H), 2,13 s und 2,09 s (zusammen 3 H), 1,38, 1,25, 1,28 und 1,07 ppm s (zusammen 6 H).

b) 22,5 g (0,132 mol) 3-Acetoxymethyl-2,2-dimethylcyclobutanon werden in 100 ml Tetrachlorkohlenstoff bei 0 bis 10°C unter Rühren tropfenweise mit 22,0 g (0,137 mol) Brom in 50 ml Tetrachlorkohlenstoff versetzt. Nach Stehen über Nacht wäscht man die Reaktionslösung neutral, trocknet über wasserfreiem Natriumsulfat und engt ein. Man erhält 32,4 g rohes 3-Acetoxymethyl-4-brom-2,2-dimethylcyclobutanon (gleiche Isomerenverteilung wie bei dem unter a) erhaltenen Bromketon).

Beispiel 13

Herstellung von 4-Brom-3-(p-tolylsulfonylmethyl)-2,2-dimethylcyclobutanon

26,6 g (0,1 mol) 3-(p-Tosylmethyl)-2,2-dimethyl-cyclobutanon in 250 ml Ameisensäureethylester werden bei 20°C innerhalb 1 h portionsweise mit insgesamt 32,0 g (0,1 mol) Pyridiniumperbromid versetzt. Nach 5 h Nachrühren gießt man den Ansatz auf Eiswasser, trennt die Phasen, wäscht die organische Phase neutral und klärt sie über wasserfreiem Natriumsulfat. Einengen i. Vak. liefert 33,6 g rohes 4-Brom-3-(p-tosylmethyl)-2,2-dimethyl-cyclobutanon als Stereoisomerengemisch (Isomerenverhältnis 8:1). Kristallisieren aus Methanol/Essigsäuremethylester ergibt die Hauptkomponente als farblose Kristalle vom Schmp. 143-146°C.

| | | | | | |
|---|---|---|---|---|---|
| $C_{14}H_{17}BrSO_3$ | Ber. | C 48,70 | H 4,96 | Br 23,15 | S 9,29 |
| (345.3) | Gef. | 48,75 | 4,9 | 23,9 | 9,65 |

IR ($CCl_4$): 1800 $cm^{-1}$

$^1$H-NMR ($CDCl_3$): $\delta$ = 7,85 und 7,43 $A_2B_2$-Typ (4 H, J = 9 Hz), 4,92 d (1 H, J = 8 Hz), 3,48 d (2 H, J = 7 Hz), 2,63 mc (1 H), 2,47 s (3 H), 1,35 s (3 H) und 1,25 ppm s (3 H).

Beispiel 14

4-Brom-3-methoxymethyl-2,2-dimethyl-cyclobutanon

18,0 g (0,126 mol) 3-Methoxymethyl-2,2-dimethyl-cyclobutanon werden in 190 ml Essigsäureethylester mit 40,2 g (0,126 mol) Pyridiniumperbromid, wie in Beispiel 12 beschrieben, umgesetzt. Man erhält 24,0 g rohes Bromketon, dessen fraktionierende Destillation 15,7 g 4-Brom-3-methoxymethyl-2,2-dimethyl-cyclobutanon vom $Sdp._{0,1}$ 52-54°C liefert (Isomerengemisch, Verhältnis 13:3).

| | | | | |
|---|---|---|---|---|
| $C_8H_{13}BrO_2$ | Ber. | C 43,46 | H 5,93 | Br 36,14 |
| (221.1) | Gef. | 43,4 | 5,88 | 35,9 |

IR ($CCl_4$): 1800 $cm^{-1}$

$^1H$-NMR ($CDCl_3$): $\delta$ = 5,21 und 4,85 d (zusammen 1 H, J = 9 bzw. 8 Hz), 3,67 d (2 H, J = 7 Hz), 3,39 s (3 H), 2,50 mc (1 H), 1,32, 1,25 und 1,21 ppm s (zusammen 6 H).

Beispiel 15

4-Brom-3-chlormethyl-2,2-dimethyl-cyclobutanon

30,6 g (0,21 mol) 3-Chlormethyl-2,2-dimethyl-cyclobutanon werden in 300 ml Essigsäureethylester, wie in Beispiel 12 beschrieben, mit 67,9 g (0,21 mol) Pyridiniumperbromid umgesetzt. Nach üblichem Aufarbeiten werden 45,1 g rohes Bromketon erhalten, die fraktionierend destilliert werden. 32,3 g 4-Brom-3-chlormethyl-2,2-dimethyl-cyclobutanon vom $Sdp._{0,08}$ 63-67°C werden erhalten (Stereoisomerenverhältnis 14:3). Kristallisieren aus n-Hexan liefert das Isomer vom Schmp. 53,5-55°C.

| | | | | | |
|---|---|---|---|---|---|
| $C_7H_{10}BrClO$ | Ber. | C 37,28 | H 4,47 | Br 35,44 | Cl 15,72 |
| (225.5) | Gef. | 37,0 | 4,33 | 35,0 | 16,1 |

IR ($CCl_4$): 1800 $cm^{-1}$

$^1$H-NMR ($CDCl_3$): $\delta$ = 4,80 d (1 H, J = 8 Hz), 3,85 mc (2 H), 2,65 mc (1 H), 1,40 s (3 H) und 1,30 ppm s (3 H).

Beispiel 16

4-Brom-3-brommethyl-2,2-dimethyl-cyclobutanon

26,3 g (0,137 mol) 3-Brommethyl-2,2-dimethyl-cyclobutanon werden in 200 ml Essigsäureethylester mit 44,0 g (0,137 mol) Pyridiniumperbromid wie in Beispiel 12 beschrieben, umgesetzt. Nach 48 h Stehen bei 20°C arbeitet man wie angegeben auf und erhält 35,5 g rohes Bromketon (Isomerengemisch, Verhältnis 15:2). Kristallisieren aus n-Hexan liefert das Isomer vom Schmp. 61-62°C.

| | | | | |
|---|---|---|---|---|
| $C_7H_{10}Br_2O$ | Ber. | C 31.14 | H 3,73 | Br 59,20 |
| (270.0) | Gef. | 31,0 | 3,67 | 60,1 |

IR ($CCl_4$): 1800 $cm^{-1}$

$^1$H-NMR ($CDCl_3$) $\delta$ = 4,75 d (1 H, J = 8 Hz), 3,68 mc (2 H), 2,75 mc (1 H), 1,40 s (3 H) und 1,30 ppm s (3 H).

Beispiel 17

4-Brom-3-cyanmethyl-2,2-dimethyl-cyclobutanon

13,6 g (0,1 mol) 3-Cyanmethyl-2,2-dimethyl-cyclobutanon in 200 ml Essigsäuremethylester werden innerhalb von 2 h mit 32,0 g 0,1 mol Pyridiniumperbromid bei 20°C versetzt. Nach 24 h Rühren bei 20°C gießt man das Gemisch in 200 g Eis, trennt die Phasen, trocknet die org. Phase über wasserfreiem Natriumsulfat und engt nach Filtrieren i. Vak. ein. Den Rückstand (20,7 g) destilliert man fraktionierend i. Vak. und erhält 10,6 g 4-Brom-3-cyanmethyl-2,2-dimethyl-cyclobutanon vom $Sdp._{0,1-0,18}$ 97-105°C (Isomerengemisch, Verhältnis 17:5).

| | | | | |
|---|---|---|---|---|
| $C_8H_{10}BrNO$ | Ber. | C 44,47 | H 4,67 | Br 36,98 |
| (216.1) | Gef. | 44,2 | 4,90 | 36,4 |

IR ($CCl_4$): 1800 und 2130 $cm^{-1}$

$^1H$-NMR ($CDCl_3$): $\delta$ = 5,38 und 4,93 d (zusammen 1 H, J = 9 bzw. 8 Hz), 2,75 mc (3 H), 1,44, 1,40, 1,38 und 1,35 ppm s (zusammen 6 H).

Beispiel 18

3-Acetoxymethyl-2,2-dimethyl-cyclopropan-carbonsäure

32,4 g (~0,13 mol) rohes 3-Acetoxymethyl-4-brom-2,2-dimethylcyclobutanon werden in 100 ml 2 n NaOH bei 20°C gerührt. Nach 3-5 h resultiert eine homogene Lösung. Man extrahiert Neutralprodukte mit Ether, säuert die wäßrig-alkalische Lösung unter Kühlen mit konz. Salzsäure an und extrahiert erschöpfend mit Ether. Trocknen über wasserfreiem Natriumsulfat und Einengen i. Vak. liefert 18,2 g 3-Acetoxymethyl-2,2-dimethyl-cyclopropancarbonsäure.

Beispiel 19

2,2-Dimethyl-3-hydroxymethyl-cyclopropancarbonsäure

32.1 g (~0,13 mol) rohes 3-Acetoxymethyl-4-brom-2,2-dimethylcyclobutanon werden in 300 ml 2n NaOH 2 h bei 30-40°C und anschließend über Nacht bei 20°C gerührt. Man extrahiert neutrale Produkte (0,74 g) mit Ether, säuert die wäßrig-alkalische Lösung mit Salzsäure unter Kühlen an und extrahiert mit Ether. Trocknen des Etherextraktes über wasserfreiem Magnesiumsulfat und Einengen i. Vak. liefert 12,0 g 3-Hydroxymethyl-2,2-dimethyl-cyclopropancarbonsäure als farbloses Öl.

Durch erschöpfendes Extrahieren in einer Kutscher-Steudel-Apparatur mit Ether werden wietere 3,4 g der recht gut wasserlöslichen Säure erhalten.

Analog Beispiel 19 sind erhalten worden:

1. 2,2-Dimethyl-3-methoxymethyl-cyclopropancarbonsäure, Sdp. $_{0,2}$ 160-170°C (Kugelrohr).

2. 2,2-Dimethyl-3-p-tosylmethyl-cyclopropancarbonsäure, Schmp. 172-174°C (aus Chloroform/Ether).

Beispiel 20

2,2-Dimethyl-3-hydroxymethyl-cyclopropancarbonsäuremethylester

Zu der Lösung von 2,3 g (0,1 g-atom) Natrium in 100 ml abs. Methanol tropft man unter Feuchtigkeitsausschluß und Kühlung 24,9 g (0,1 mol) 3-Acetoxymethyl-4-brom-2,2-dimethyl-cyclobutanon in 50 ml abs. Methanol und erhitzt anschließend 3 h zum Rückfluß. Nach Eindampfen i.Vak. löst man den Rückstand (26.0 g) in eiskaltem Wasser und extrahiert die schwach alkalische Lösung mit Ether (Kutscher-Steudel-Apparatur). Trocknen der org. Phase über wasserfreiem Natriumsulfat und Einengen liefern 14,1 g schwachgelb gefärbtes rohen 2,2-Dimethyl-3-hydroxymethyl-cyclopropancarbonsäure-methylester, identisch mit einem durch Veresterung von roher 3-Hydroxymethyl-2,2-dimethyl-cyclopropancarbonsäure (Beispiel 19) mit Diazomethan erhaltenen Präparat.

0051791

Patentansprüche

1. Verbindungen der Formel I

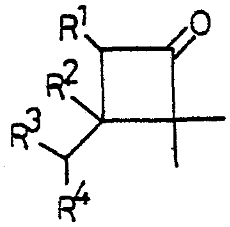

(I)

worin

$R^1$ für Wasserstoff, Chlor oder Brom,

$R^2$ für Wasserstoff, Methyl oder Phenyl,

$R^3$ für Wasserstoff, Alkyl, Aralkyl, Aryl, Halomethyl, Acyloxymethyl oder den Rest P(O)(O Alkyl)$_2$ steht und

$R^4$ für Chlor, Brom, Cyan, Alkoxy, Aryloxy, Acyloxy, Acylmercapto, Arylsulfonyl, Alkylsulfonyl, Acylamino oder P(O) (O Alkyl)$_2$ steht.

2. Verfahren zur Herstellung von Verbindungen der Formel I

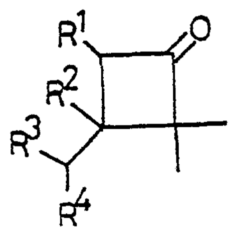

(I)

worin

$R^1$ für Wasserstoff, Chlor oder Brom,

$R^2$ für Wasserstoff, Methyl oder Phenyl,

$R^3$ für Wasserstoff, Alkyl, Aralkyl, Aryl, Halomethyl, Acyloxymethyl oder den Rest (P(O)(O Alkyl)$_2$ steht und

$R^4$ für Chlor, Brom, Cyan, Alkoxy, Aryloxy, Acyloxy, Acylmercapto, Arylsulfonyl, Alkylsulfonyl, Acylamino oder P(O) (O Alkyl)$_2$ steht

dadurch gekennzeichnet, daß man terminale Olefine der Formel II

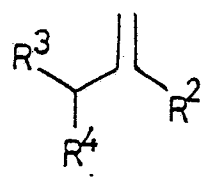

(II)

mit 1-Chlor-1-dimethylamino-2-methylpropen-1 und einer Lewis-Säure, vorzugsweise Zinkchlorid, bei 20-40°C umsetzt und das Reaktionsprodukt anschließend hydrolysiert und gegebenenfalls mit einem Halogenierungsmittel umsetzt.

Europäisches Patentamt

# EUROPÄISCHER RECHERCHENBERICHT

0051791

Nummer der Anmeldung

EP 81 10 8956

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | DE - A - 2 539 048 (SHELL INTERNATIONALE RESEARCH)<br>* Seite 25, Zeilen 28,29 *<br>-- | 1 | C 07 C 49/457<br>49/517<br>69/14<br>69/16<br>69/63<br>103/42<br>121/46<br>147/02<br>147/06<br>153/09<br>C 07 F 9/40<br>C 07 C 45/51<br>45/63<br>67/287<br>120/00 ./. |
| X | DE - A - 2 638 356 (BAYER)<br>* Seite 3, Zeile 20 - Seite 4, Zeile 16; Seite 5, Zeile 15 - Seite 6, Zeile 15; Seite 24, Zeilen 3-6; Seite 25, Zeilen 1-4; Seite 10, Zeilen 5-10; Seite 19, Zeile 18 - Seite 20, Zeile 18 *<br>---- | 1,2 | RECHERCHIERTE SACHGEBIETE (Int. Cl.³)<br>C 07 C 45/51<br>49/457<br>49/517<br>67/293<br>69/14<br>69/16<br>69/63 |
| | | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 03-02-1982 | WRIGHT |

Europäisches Patentamt

**EUROPÄISCHER RECHERCHENBERICHT**

0051791

Nummer der Anmeldung

EP 81 10 8956

-2-

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der Maßgeblichen Teile | betrifft Anspruch | |
| | | | C 07 C 62/02<br>62/08<br>69/757<br>51/09<br>67/475 |
| | | | RECHERCHIERTE SACHGEBIETE (Int. Cl.3) |
| | | | |